# EUROPEAN PATENT SPECIFICATION

(11) **EP 4 306 951 B1**
(45) Date of publication and mention of the grant of the patent: **31.12.2025**
(21) Application number: 23184879.7
(22) Date of filing: 11.07.2023
(51) Int. Cl.: G01N 33/00, G01N 33/28, G01N 33/30, G01N 27/12, G01N 27/22

(54) **LUBRICANT MONITORING**
SCHMIERMITTELÜBERWACHUNG
SURVEILLANCE DE LUBRIFIANT

(30) Priority: 12.07.2022 GB 202210227
(43) Date of publication of application: 17.01.2024
(73) Proprietor: ESR Technology Limited, Warrington Cheshire WA3 6WU (GB)
(72) Inventor: RENONDEAU, Hugues, Warrington, WA3 6WU (GB)
(74) Representative: Mansfield, Peter Turquand

(56) References cited:
- JP-A- 2020 041 810
- FUKUSHIMA Y ET AL: "Combined Effect of Phosphate Ester and OBCS on Tribochemical Decomposition of Hydrocarbon Oil on Nascent Steel Surfaces", TRIBOLOGY LETTERS, SPRINGER US, NEW YORK, vol. 63, no. 1, 19 May 2016 (2016-05-19), pages 1 - 8, XP035689290, ISSN: 1023-8883, [retrieved on 20160519], DOI: 10.1007/S11249-016-0687-6
- JOHN P J ET AL: "Tribological behavior of a multialkylated cyclopentane oil under ultrahigh vacuum conditions", TRIBOLOGY LETTERS, 1 January 2001 (2001-01-01), Dordrecht, pages 167 - 173, XP093106859, Retrieved from the Internet <URL:https://link.springer.com/article/10.1023/A:1018808921623> [retrieved on 20231129], DOI: 10.1023/A:1018808921623

## Description

The invention relates to an apparatus and a method for monitoring lubricant within a low-pressure environment, such as within a vacuum system, to detect deterioration of the lubricant before there is a consequential increase in friction of the lubricated mechanism.

The production of many advanced components for scientific instruments, semi-conductors, photovoltaic, transport and other industrial sectors requires the use of vacuum processing using an evacuated chamber usually achieved using mechanical pumps. Presently in the vacuum pump industry, maintenance schedules are generally based on pump running time, which can cause pumps to be serviced at unsuitable intervals costing time and higher than necessary maintenance costs if the pumps are still operating at nominal status. However, many industrial vacuum pumps may extract reactive gases, which can interact with lubricants to accelerate degradation; other pumps are used in a highly intermittent way with many stop/starts which is a tribologically challenging operational mode. Therefore service intervals are set by manufacturers conservatively to ensure warranty claims by these outlying applications are not excessive. This means there is a possibility that a high number of pumps operating in "nominal" use cases may be serviced more frequently than justified by their lubricant health status, with associated higher-than optimal maintenance costs. Furthermore, if service is not correctly carried out or if some system or external issue/fault causes elevated temperature for example, the tribological elements within the system rapidly come under operational stress (high load due to misalignment or incorrect preload/service/poor lubrication etc). So a way of monitoring the state of the lubricant in such a vacuum system during operation would be beneficial if it could detect lubricant deterioration before the deterioration is such as to affect friction within a lubricated bearing, and ultimately bearing failure.

An article by Fukushima et al. (Tribology Letters (2016) 63:3) describes use of multi-alkylated cyclopentane (MAC) as a lubricant suitable for use in space, because of its low vapour pressure, but that it decomposes on contact with metal to give small molecules such as H₂, CH₄, and C₂H₆. Certain additives can reduce this decomposition. Tribology tests were carried out using a quadrupole mass spectrometer to observe the gas molecules, and the effects of additives were observed.

JP2020/041810 (NSK Ltd) describes using a gas sensor to detect a polar gas generated on deterioration of a lubricant. The gas sensor may be a quartz crystal microbalance, or a surface stress sensor, or a surface acoustic wave sensor; in each case a film of ionic liquid is provided on its surface, such as 1-butyl-3-methylimidazolium chloride. The sensor can detect polar molecules such as alcohols and carbonyl compounds; for example n-heptanal, acetic acid, and n-heptanol can be detected.

An article by P. J. John et al. (Tribology Letters Vol. 9, No. 3-4, 2000) describes use of MAC as a lubricant for use in space. Tests were carried out with a ball-on-flat tribometer and different lubricant conditions, finding the lubricant lifetime markedly less in a vacuum than in a nitrogen environment, presumably due to evaporation effects. A quadrupole mass spectrometer was used to study gases produced during use of the tribometer, and CH₃ and CH₄ were observed.

The scope of the present invention is defined in the claims.

According to the present invention there is provided a lubricant monitor suitable for use within a low pressure environment in a vacuum system or in a satellite in space, the monitor comprising a gas-phase sensor operable to detect at least one chemical compound created as a consequence of lubricant deterioration within the low pressure environment and to monitor the variation in the detected quantity of that compound, the detected quantity being indicative of the rate of formation of that compound during operation, and thereby to detect lubricant deterioration before it has detrimental frictional effects, wherein if the lubricant comprises PFPE, the monitored compound is COF, COF₂, CF₃, C₂F₅ or C₂OF₅, and if the lubricant comprises multiply-alkylated cyclopentane (MAC), the monitored compound is C₃H₇, C₆H₆ or CO₂, and the monitor detecting the initial substantially steady rate of formation of the compound, and detecting when the compound is formed at a much greater rate as being an indication of deterioration of the lubricant.

Thus the monitor detects gases or vapours in a location near to but spaced away from the lubricant, where that location is evacuated or communicates freely with a vacuum system. As chemical compounds are formed and evaporate, they diffuse out, past the sensing location, into the vacuum system. The monitored concentration of a chemical compound is hence a measure of the rate at which that compound is being formed from the lubricant. The low pressure environment may be created by a vacuum pump, or indeed may be the low pressure of space, for example to monitor degradation of a lubricant in an artificial satellite.

The gas-phase sensor may for example be a resistive sensor or a work function sensor. A resistive sensor is a semiconducting metal-oxide or metallic resistor device. The electrical properties of metal oxides change when they are exposed to reducing gases, and this can be exploited to detect combustible and reducing gases. Response times for these sensors are in the range 4-20s. A work function sensor is one of a range of different types: Schottky diode, MOS field effect transistor, MIS capacitor. These sensors generally have a triple layer metal, oxide, and semiconductor structure. The work function is the minimum energy required to remove an electron from a solid surface to infinity, and in these sensors the work function is altered by gas exposure and a measurable voltage change is produced. Schottky diode types operate at <250°C and have response times of 1s. MIS capacitor types have response time of 20-50s however this is dependent on gas concentration.

It will be appreciated that some of these sensors may take several seconds to respond, but that is acceptable in the present context as the changes in the lubricant are gradual, typically occurring over long periods, for example of hours.

In a second aspect, the invention provides a method of monitoring a lubricant within a low pressure environment such as a vacuum system or a satellite in space, by operating a gas-phase sensor within the low pressure environment to detect at least one chemical compound created as a consequence of lubricant deterioration and to monitor the variation in the quantity of that compound during operation of the low pressure environment, and thereby to detect lubricant deterioration before it has detrimental frictional effects.

The invention monitors for degradation products released from the lubricants within the pump before tribological failure occurs, providing an advance warning and giving end-users an overview of their entire system performance. The invention enables incipient detection of end-of-life of the lubricant before a bearing catastrophically fails. This is clearly very beneficial, enabling catastrophic bearing failure to be prevented, while enabling maintenance to be carried out when it is actually required. Indeed the use of such a sensor in association with IoT (Internet of Things) technology which is already being used by some manufacturers could permit service needs to be assessed by the pump operator or pump manufacturer's own service team (or even an autonomous expert system) on the basis of lubricant health.

It has been found that the chemical compounds arising from lubricant deterioration are initially formed at a fairly steady rate, but that surprisingly they are formed at a much greater rate just as or just before there is a significant deterioration of the lubricant. Hence that imminent deterioration can be anticipated by detecting the rate of formation of at least one such compound. For example the monitor may continuously determine the average (over time) of the monitored concentration of the sensed compound, and warn of lubricant deterioration if the observed level is more than n times the average, where n is at least 4, and may be 5 or 6 or more.

The invention will now be further and more particularly described by way of example only and with reference to the accompanying drawings in which:
Figure 1 shows graphically the variations of the coefficient of friction and of partial pressures of several different compounds during a rolling test on a perfluoropolyether (PFPE) lubricant, using a residual gas analyser;
Figure 2 shows graphically the variations of the coefficient of friction and the partial pressure of the compound CF₃ (molar mass 69) during a rolling test on a PFPE lubricant, measured with a residual gas analyser;
Figure 3 shows graphically the variations of the coefficient of friction and the partial pressure of a small-chain hydrocarbon compound during a rolling test on a multiply-alkylated cyclopentane (MAC) lubricant, using a residual gas analyser; and
Figure 4 shows a schematic diagram of a lubricant monitor of the invention.

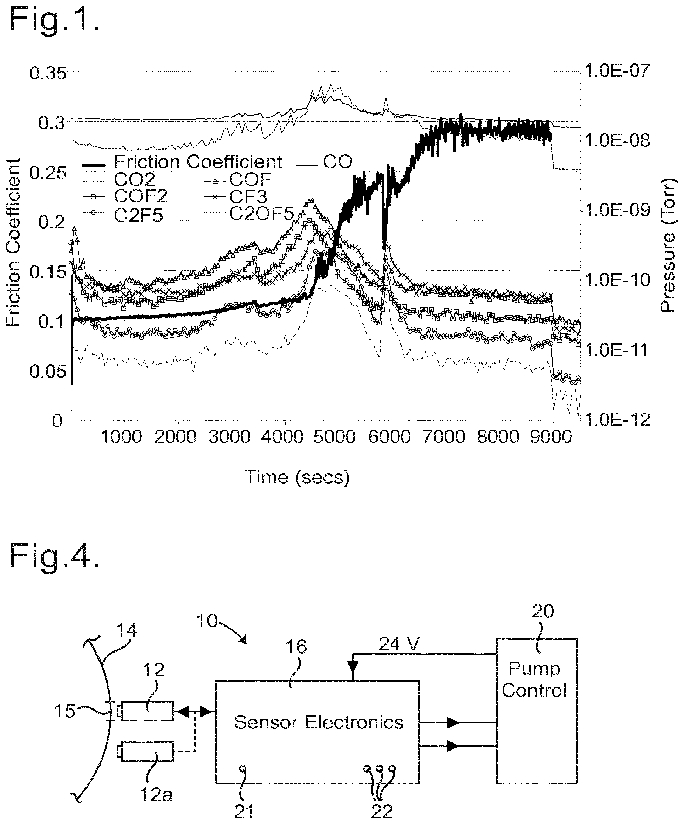

Referring now to Figure 1, this shows measurements taken during an experiment using a spiral orbit tribometer in conjunction with a quadrupole residual gas analyzer (RGA). A 12.7mm 440C steel test ball was lubricated with 815Z oil (equivalent to Fomblin Z25), inserted between 440C steel plates and evacuated to 1.5 x 10⁻⁸ Torr (usual test procedure), at room temperature. This oil is a PFPE lubricant commonly used in vacuum equipment. Motion of the spiral orbit tribometer was then started, with the test running until failure occurred in the usual manner, and in this case the test run was continued after lubricant failure had been observed. The coefficient of friction is initially about 0.10 and it gradually increases to about 0.12 after about 4500 s; at that point the lubricant begins to seriously deteriorate, the coefficient of friction increasing rapidly to above 0.20, and then rising again to a value of about 0.3. The RGA was used to monitor several different compounds, each of molar mass less than 150 AMU. The graph shows the variations of partial pressure of several different compounds believed to be related to the degradation and cracking of the PFPE (namely CO 28 AMU, CO₂ 44 AMU, COF 47 AMU, COF₂ 66 AMU, CF₃ 69 AMU, C₂F₅ 119 AMU and C₂OF₅ 135 AMU). However it is noted that at 28 AMU CO shares a weight with N₂, and that this, along with CO₂, will be present in normal laboratory air. It is therefore a possibility that the measured partial pressure at this AMU may be a combination of CO and N₂, giving a false high value in comparison to the gas emitted as a result of the oil degradation.

In every case the partial pressure of each compound gradually increases during the rolling test, in each case increasing to a peak and then decreasing (it will be appreciated that the pressure axis scale is logarithmic). The peak occurs just before or just as the friction coefficient starts to increase rapidly. The nature of the spiral orbit tribometer (SOT) ensures that all the applied lubricant is at the same state of degradation at a given time. The peak emission of all these compounds suggests that the increase in friction (i.e. decrease in lubrication) is due to chemical breakdown of the lubricant; and the subsequent decrease in the partial pressures of all these compounds is because much of the lubricant has been destroyed. It seems reasonable to conclude that the primary mode of failure of the lubricant is due to tribo-chemical change, that is to say chemical breakdown due to frictional effects.

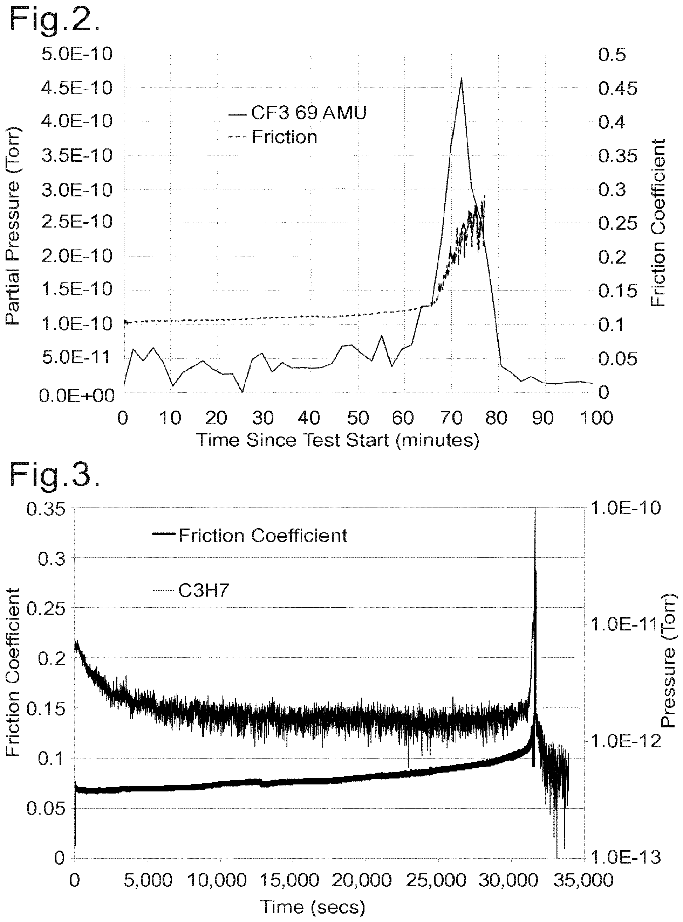

Referring now to Figure 2 there are shown results from a similar experiment, measurements being taken during an experiment using a spiral orbit tribometer (SOT) in conjunction with a quadrupole residual gas analyzer (RGA). In this case only the partial pressure figures for CF3 (69 AMU) are shown. Again there is a dramatic peak in the measured compound just as the coefficient of friction starts to increase rapidly; in this case the pressure axis is linear. The height of the peak is almost ten times the average value prior to the peak. This indicates that chemical degradation occurs, and the ineffectual degraded lubricant results in the increase in friction.

Another type of lubricant suitable for use in a low-pressure environment is a multiply-alkylated cyclopentane (MAC). This type of lubricant also deteriorates over prolonged use, though in a somewhat different way to the PFPE lubricant described above. MAC degradation is driven by the decomposition of the core lubricant molecule into long chain hydrocarbons. These hydrocarbons are then sheared, within a tribological contact, and experience "cracking". This process results in smaller chain hydrocarbons, which are then able to polymerise to form much larger hydrocarbons than the original molecules. When a critical mass of the larger molecules is reached then the lubricant is no longer able to provide sufficient lubricity and the friction increases to failure.

Referring now to Figure 3 there are shown results from an experiment using Nye 2001 oil, a MAC lubricant, measurements being taken during an experiment using a spiral orbit tribometer (SOT) in conjunction with a quadrupole residual gas analyzer (RGA), in an evacuated enclosure. The SOT uses a 440C steel ball of diameter 12.7 mm between 440C steel plates. The partial pressure figures for C₃H₇ (55 AMU) are shown, the pressure axis being logarithmic. The pressure initially decreases due to the initial drop in chamber pressure, and then is substantially constant for a prolonged period; there is then a very significant peak in the measured compound just as the coefficient of friction starts to increase rapidly. The height of the peak is about a hundred times the average value in the prolonged period prior to the peak. Detection of such a peak is a warning that the lubricant has deteriorated and that the friction coefficient is about to increase significantly.

It will be appreciated that other small molecular weight compounds may be tracked instead, for example C₆H₆ (78 AMU), or CO₂ (44 AMU), both these compounds being particularly produced as the lubricant deteriorates.

It will be appreciated that a residual gas analyser (RGA), although suitable for experimental work, is a comparatively sophisticated and expensive piece of apparatus. The experimental data described above indicates how a range of compounds are produced as a result of tribo-chemical effects on the lubricant; but the present invention provides more economical ways to obtain such measurements, monitoring at least one such compound whose presence is indicative of deterioration of the lubricant.

Referring to Figure 3, this shows a diagrammatic view of a lubricant monitor 10 of the invention. The monitor 10 comprises a sensor head 12 which is interfaced to the vacuum pump/system 14 (indicated schematically) through a defined vacuum port 15, which may be of diameter 20-30mm. The sensor head 12 is connected to a sensor electronics unit 16 via a dedicated electrical power/data harness line. The electronics unit 16 is powered from a 24V supply provided from a vacuum pump control system 20. In this embodiment, the electronics unit 16 can be interfaced to any one of several potential sensor head types (selected to be compatible with the lubricant chemistry to be monitored); one such different sensor head 12a is shown. The electronics unit 16 provides visual indicators: an indicator 21 of system status, and indicators 22 of lubricant health (using a traffic light system). In addition it provides electronic output signals to the vacuum pump control system 20: a sensor status (OK/Not OK "handshake") output; and an analogue output signal to the pump control system, which can log and analyse the analogue data provided, together with data from other pump sensors (pressure, temperature, etc) and take action according to some criteria, for example upon reaching a specified degradation concentration (e.g. turn off/disable pump), or provide a "maintenance due" output signal in suitable form to a local facility condition monitoring system (and/or an "internet of things" based system).

By way of example the sensor head 12 may incorporate a resistive sensor which is sensitive to the presence of CF₃ in the gas phase within the vacuum pump/system 14. As described above in relation to Figures 1 and 2, with a PFPE lubricant in the vacuum pump/system 14 one will observe CF₃ in the gas phase, and this reaches a peak just as the friction starts to significantly increase. Hence by detecting the marked increase - for example detecting when the value is five times greater than the average up to that point - the imminent increase in friction can be reliably predicted, and appropriate action taken prior to failure of the lubricant, preventing damage to the bearing.

Thus the invention enables incipient failure of oils and greases used in vacuum equipment to be identified prior to significant increase in friction by monitoring the constituents (key chemical markers) of the local environment for indicators of lubricant degradation. The invention thus provides a lubricant "health sensor" system, which uses such measurements to provide advanced warning of tribological problems during operation of vacuum pumps (or other vacuum systems/equipment), permitting maintenance action before costly hardware failure and downtime.

## Claims

1. A lubricant monitor, the monitor (10) detecting at least one chemical compound created as a consequence of lubricant deterioration, wherein the lubricant is in a low pressure environment in a vacuum system or in a satellite in space, and the monitor (10) comprises a gas-phase sensor (12, 12a) suitable for use within the low pressure environment to monitor the variation in the quantity of that detected compound during operation, and thereby to detect lubricant deterioration occurring in the low pressure environment before it has detrimental frictional effects, wherein if the lubricant comprises PFPE, the monitored compound is COF, COF₂, CF₃, C₂F₅ or C₂OF₅, and if the lubricant comprises multiply-alkylated cyclopentane (MAC), the monitored compound is C₃H₇, C₆H₆ or CO₂, and the monitor (10) being configured to detect the initial substantially steady rate of formation of the compound, **characterised in that** the monitor (10) is further configured to detect when the compound is formed at a much greater rate as being an indication of deterioration of the lubricant.

2. A lubricant monitor (10) as claimed in claim 1 wherein the gas-phase sensor (12, 12a) is a resistive sensor or a work function sensor.

3. A lubricant monitor (10) as claimed in claim 2 wherein the gas-phase sensor (12, 12a) is a semiconducting metal-oxide or metallic resistor device.

4. A lubricant monitor (10) as claimed in claim 2 wherein the gas-phase sensor (12, 12a) is selected from: a Schottky diode, an MOS field effect transistor, or an MIS capacitor.

5. A lubricant monitor (10) as claimed in any one of the preceding claims wherein the monitor (10) is arranged to continuously determine the average (over time) of the monitored concentration of the sensed compound, and to warn of lubricant deterioration if the monitored concentration is more than n times the average, where n is at least 4.

6. A method of monitoring a lubricant by detecting at least one chemical compound created as a consequence of lubricant deterioration, wherein the lubricant is in a low pressure environment in a vacuum system or in a satellite in space, and the method comprises operating a gas-phase sensor (12, 12a) within the low-pressure environment, to monitor the variation in the quantity of that compound during operation, and thereby to detect lubricant deterioration occurring within the low-pressure environment before it has detrimental frictional effects, wherein if the lubricant comprises PFPE, the monitored compound is COF, COF₂, CF₃, C₂F₅ or C₂OF₅, and if the lubricant comprises multiply-alkylated cyclopentane (MAC), the monitored compound is C₃H₇, C₆H₆ or CO₂, and the method comprises detecting the initial substantially steady rate of formation of the compound, **characterised in that** the method further comprises detecting when the compound is formed at a much greater rate as being an indication of deterioration of the lubricant.

7. A lubricant monitoring method as claimed in claim 6 wherein the gas-phase sensor is a resistive sensor or a work function sensor.

8. A lubricant monitoring method as claimed in claim 7 wherein the gas-phase sensor is a semiconducting metal-oxide or metallic resistor device.

9. A lubricant monitoring method as claimed in claim 7 wherein the gas-phase sensor is selected from: a Schottky diode, an MOS field effect transistor, or an MIS capacitor.

10. A lubricant monitoring method as claimed in any one of claims 6 to 9 comprising continuously determining the average (over time) of the monitored concentration of the sensed compound, and warning of lubricant deterioration if the monitored concentration is more than n times the average, where n is at least 4.

## Patentansprüche

1. Ein Schmiermittelüberwachungsgerät, wobei das Überwachungsgerät (10) mindestens eine infolge von Schmiermittelzersetzung entstandene chemische Verbindung detektiert, wobei das Schmiermittel sich in einer Niederdruckumgebung in einem Vakuumsystem oder in einem Satelliten im Weltraum befindet und das Überwachungsgerät (10) einen Gasphasensensor (12, 12a) umfasst, der zur Verwendung innerhalb der Niederdruckumgebung geeignet ist, um die Variation der Quantität dieser detektierten Verbindung während des Betriebs zu überwachen, und dadurch die in der Niederdruckumgebung stattfindende Schmiermittelzersetzung zu detektieren, bevor sie schädliche Reibungseffekte hat, wobei, wenn das Schmiermittel PFPE umfasst, die überwachte Verbindung COF, COF₂, CF₃, C₂F₅ oder C₂OF₅ ist, und wenn das Schmiermittel mehrfach alkyliertes Cyclopentan (MAC) umfasst, die überwachte Verbindung C₃H₇, C₆H₆ oder CO₂ ist, und wobei das Überwachungsgerät (10) dazu ausgelegt ist, die anfängliche im Wesentlichen gleichbleibende Bildungsrate der Verbindung zu detektieren, **dadurch gekennzeichnet, dass** das Überwachungsgerät (10) ferner dazu ausgelegt ist, zu detektieren, wenn die Verbindung mit einer sehr viel höheren Rate gebildet wird, als einen Hinweis auf eine Zersetzung des Schmiermittels.

2. Schmiermittelüberwachungsgerät (10) nach Anspruch 1, wobei der Gasphasensensor (12, 12a) ein Widerstandssensor oder ein Austrittsarbeitssensor ist.

3. Schmiermittelüberwachungsgerät (10) nach Anspruch 2, wobei der Gasphasensensor (12, 12a) ein halbleitendes Metalloxid- oder Metall-Widerstandsgerät ist.

4. Schmiermittelüberwachungsgerät (10) nach Anspruch 2, wobei der Gasphasensensor (12, 12a) aus den Folgenden ausgewählt ist: einer Schottky-Diode, einem MOS-Feldeffekttransistor oder einem MIS-Kondensator.

5. Schmiermittelüberwachungsgerät (10) nach einem der vorhergehenden Ansprüche, wobei das Überwachungsgerät (10) dazu eingerichtet ist, kontinuierlich den Durchschnitt (im Zeitverlauf) der überwachten Konzentration der erfassten Verbindung zu bestimmen und vor einer Schmiermittelzersetzung zu warnen, wenn die überwachte Konzentration mehr als das n-Fache des Durchschnitts beträgt, wobei n mindestens 4 ist.

6. Ein Verfahren zum Überwachen eines Schmiermittels durch Detektieren mindestens einer infolge von Schmiermittelzersetzung entstandenen chemischen Verbindung, wobei sich das Schmiermittel in einer Niederdruckumgebung in einem Vakuumsystem oder in einem Satelliten im Weltraum befindet und das Verfahren das Betreiben eines Gasphasensensors (12, 12a) innerhalb der Niederdruckumgebung umfasst, um die Variation der Quantität dieser Verbindung während des Betriebs zu überwachen und dadurch die in der Niederdruckumgebung stattfindende Schmiermittelzersetzung zu detektieren, bevor sie schädliche Reibungseffekte hat, wobei, wenn das Schmiermittel PFPE umfasst, die überwachte Verbindung COF, COF₂, CF₃, C₂F₅ oder C₂OF₅ ist, und wenn das Schmiermittel mehrfach alkyliertes Cyclopentan (MAC) umfasst, die überwachte Verbindung C₃H₇, C₆H₆ oder CO₂ ist, und wobei das Verfahren das Detektieren der anfänglichen im Wesentlichen gleichbleibenden Bildungsrate der Verbindung umfasst, **dadurch gekennzeichnet, dass** das Verfahren ferner das Detektieren, wenn die Verbindung mit einer sehr viel höheren Rate gebildet wird, als einen Hinweis auf die Zersetzung des Schmiermittels umfasst.

7. Schmiermittelüberwachungsverfahren nach Anspruch 6, wobei der Gasphasensensor ein Widerstandssensor oder ein Austrittsarbeitssensor ist.

8. Schmiermittelüberwachungsverfahren nach Anspruch 7, wobei der Gasphasensensor ein halbleitendes Metalloxid- oder Metallwiderstandsgerät ist.

9. Schmiermittelüberwachungsverfahren nach Anspruch 7, wobei der Gasphasensensor aus den Folgenden ausgewählt ist: einer Schottky-Diode, einem MOS-Feldeffekttransistor oder einem MIS-Kondensator.

10. Schmiermittelüberwachungsverfahren nach einem der Ansprüche 6 bis 9, das das kontinuierliche Bestimmen des Durchschnitts (im Zeitverlauf) der überwachten Konzentration der erfassten Verbindung und das Warnen vor einer Schmiermittelzersetzung, wenn die überwachte Konzentration mehr als das n-Fache des Durchschnitts beträgt, wobei n mindestens 4 ist, umfasst.

## Revendications

1. Dispositif de surveillance de lubrifiant, le dispositif de surveillance (10) détectant au moins un composé chimique dont la création résulte d'une détérioration du lubrifiant, dans lequel le lubrifiant se trouve dans un environnement à basse pression dans un système de vide ou dans un satellite dans l'espace, et le dispositif de surveillance (10) comprenant un capteur de phase gazeuse (12, 12a) conçu pour une utilisation dans l'environnement à basse pression pour surveiller la variation de la quantité de ce composé détecté en cours de fonctionnement, et ainsi détecter la détérioration du lubrifiant se produisant dans l'environnement à basse pression avant qu'elle n'ait des effets de frottement néfastes, dans lequel si le lubrifiant comprend du PFPE, le composé surveillé est COF, COF₂, CF₃, C₂F₅ ou C₂OF₅, et si le lubrifiant comprend du cyclopentane poly-alkylé (MAC), le composé surveillé est C₃H₇, C₆H₆ ou CO₂, et le dispositif de surveillance (10) étant configuré pour détecter la vitesse initiale sensiblement stable de formation du composé,
**caractérisé en ce que** le dispositif de surveillance (10) est en outre configuré pour détecter le moment où le composé se forme à une vitesse beaucoup plus élevée, ce qui constitue une indication de détérioration du lubrifiant.

2. Dispositif de surveillance de lubrifiant (10) selon la revendication 1, dans lequel le capteur de phase gazeuse (12, 12a) est un capteur résistif ou un capteur de fonction de travail.

3. Dispositif de surveillance de lubrifiant (10) selon la revendication 2, dans lequel le capteur de phase gazeuse (12, 12a) est un dispositif semi-conducteur à oxyde métallique ou à résistance métallique.

4. Dispositif de surveillance de lubrifiant (10) selon la revendication 2, dans lequel le capteur de phase gazeuse (12, 12a) est sélectionné parmi : une diode Schottky, un transistor MOS à effet de champ ou un condensateur MIS.

5. Dispositif de surveillance de lubrifiant (10) selon l'une quelconque des revendications précédentes, le dispositif de surveillance (10) étant agencé pour déterminer en continu la moyenne (dans le temps) de la concentration surveillée du composé détecté, et pour avertir de la détérioration du lubrifiant si la concentration surveillée est supérieure à n fois la moyenne, n valant au moins 4.

6. Procédé de surveillance d'un lubrifiant par détection d'au moins un composé chimique dont la création résulte d'une détérioration du lubrifiant, dans lequel le lubrifiant se trouve dans un environnement à basse pression dans un système de vide ou dans un satellite dans l'espace, et le procédé comprenant l'utilisation d'un capteur de phase gazeuse (12, 12a) dans l'environnement à basse pression, pour surveiller la variation de la quantité de ce composé en cours de fonctionnement, et ainsi détecter la détérioration du lubrifiant se produisant dans l'environnement à basse pression avant qu'elle n'ait des effets de frottement néfastes, dans lequel si le lubrifiant comprend du PFPE, le composé surveillé est COF, COF₂, CF₃, C₂F₅ ou C₂OF₅, et si le lubrifiant comprend du cyclopentane poly-alkylé (MAC), le composé surveillé est C₃H₇, C₆H₆ ou CO₂, et le procédé comprenant une détection de la vitesse initiale sensiblement stable de formation du composé, **caractérisé en ce que** le procédé comprend en outre une détection du moment où le composé se forme à une vitesse beaucoup plus élevée, ce qui constitue une indication de détérioration du lubrifiant.

7. Procédé de surveillance de lubrifiant selon la revendication 6, dans lequel le capteur de phase gazeuse est un capteur résistif ou un capteur de fonction de travail.

8. Procédé de surveillance de lubrifiant selon la revendication 7, dans lequel le capteur de phase gazeuse est un dispositif semi-conducteur à oxyde métallique ou à résistance métallique.

9. Procédé de surveillance de lubrifiant selon la revendication 7, dans lequel le capteur de phase gazeuse est sélectionné parmi : une diode Schottky, un transistor MOS à effet de champ ou un condensateur MIS.

10. Procédé de surveillance de lubrifiant selon l'une quelconque des revendications 6 à 9, comprenant une détermination en continu de la moyenne (dans le temps) de la concentration surveillée du composé détecté, et un avertissement de la détérioration du lubrifiant si la concentration surveillée est supérieure à n fois la moyenne, n valant au moins 4.
